# EUROPÄISCHE PATENTSCHRIFT

(11) **EP 2 406 611 B1**
(45) Veröffentlichungstag und Bekanntmachung des Hinweises auf die Patenterteilung: **12.08.2015**
(21) Anmeldenummer: 10719720.4
(22) Anmeldetag: 12.03.2010
(51) Int. Cl.: G01N 11/16, G01N 33/28, F02M 25/022, F02D 19/02

(54) **VERFAHREN ZUR BESTIMMUNG DER VISKOSITÄT VON KRAFTSTOFF/WASSER- UND WASSER/KRAFTSTOFF-EMULSIONEN**
METHOD FOR DETERMINING THE VISCOSITY OF FUEL/WATER AND WATER/FUEL EMULSIONS
PROCÉDÉ PERMETTANT DE DÉTERMINER LA VISCOSITÉ D'ÉMULSIONS COMBUSTIBLE/EAU ET EAU/COMBUSTIBLE

(30) Priorität: 12.03.2009 DE 102009012446
(43) Veröffentlichungstag der Anmeldung: 18.01.2012
(73) Patentinhaber: Porep GmbH, 23775 Großenbrode (DE)
(72) Erfinder: POREP, Thomas, 23775 Großenbrode (DE)
(74) Vertreter: Lobemeier, Martin Landolf
(86) Internationale Anmeldenummer: PCT/DE2010/000266
(87) Internationale Veröffentlichungsnummer: WO 2010/102612

(56) Entgegenhaltungen:
- DE-A1-102005 012 452
- GB-A- 2 259 368
- US-A- 5 877 409
- US-A1- 2002 011 095
- US-A1- 2005 132 779
- US-A1- 2007 245 811
- GIMSON C: "USING THE CAPACITANCE CHARGE TRANSFER PRINCIPLE FOR WATER CONTENT MEASUREMENT" MEASUREMENT AND CONTROL, INSTITUTE OF MEASUREMENT AND CONTROL. LONDON, GB, Bd. 22, Nr. 3, 1. April 1989 (1989-04-01), Seiten 79-81, XP000031234 ISSN: 0020-2940

## Beschreibung

Die Erfindung betrifft ein Verfahren zur Bestimmung der Viskosität von Kraftstoff/Wasser- und Wasser/Kraftstoff-Emulsionen.

Allgemein ist bekannt, dass die Viskosität von Kraftstoffen das Förderverhalten des Kraftstoffs in den Förder- und Einspritzpumpen sowie die Zerstäubung des Kraftstoffs durch die Einspritzdüsen beeinflusst. Die Viskosität ist dabei Temperatur- und druckabhängig, wobei die Viskosität bei abnehmender Temperatur und/oder zunehmendem Druck steigt.

Eine zu hohe Viskosität führt in Kraftstoffleitungssystemen bei niedrigen Temperaturen aufgrund verminderter Pumpfähigkeit des Kraftstoffs zu Kaltstartproblemen, während eine zu niedrige Viskosität den Warmstart erschwert, zu Leistungsverlust bei hohen Temperaturen und außerdem zu Pumpenverschleiß führt.

Um die vorgenannten Probleme beim Fördern des Kraftstoffs zu vermeiden sind Kraftstoffleitungssysteme bekannt, bei denen an geeigneter Stelle eine Viskositätsmessung des Kraftstoffs durchgeführt wird, wobei in einem dem Messabschnitt vor- oder nachgeschalteten Bereich Einrichtungen zum Temperieren des Kraftstoffs vorgesehen sind, die den Kraftstoff (zumeist) durch Erhöhen der Temperatur auf eine vorbestimmte optimale Betriebstemperatur bringen und damit eine vorbestimmte optimale Viskosität des Kraftstoffs bewirken.

Derartige Systeme sind insbesondere bei Dual-Fuel-Systemen notwendig, bei denen der Betrieb der Maschine, z.B. Schiffsdieselmotoren, von einer Kraftstoffart auf eine zweite Kraftstoffart umgeschaltet wird, da sich die Kraftstoffarten regelmäßig in ihren rheologischen Eigenschaften, insbesondere ihrer Viskosität unterscheiden.

Speziell bei diesen System hat sich herausgestellt, dass die herkömmlich zur Messung der Viskosität von Kraftstoffen verwendeten Viskosimeter, insbesondere Viskosimeter, die als Sensor ein mit einer Schwingung mit hoher Frequenz und geringer Amplitude beaufschlagtes Messelement aufweisen, bei der Messung der Viskosität von Kraftstoff/Wasser- und Wasser/Kraftstoff-Emulsionen erhebliche Meßfehler aufweisen. Die Abweichungen des gemessenen Viskositätswerts vom wahren Viskositätswert liegen teilweise bei 100 % und können so leicht zu Beschädigungen der Maschinen führen.

Andere Messgeräte, wie z.B. Rotationsviskosimeter oder hochpräzise Rheometer, werden aufgrund ihres Aufbaus und der sehr hohen Anschaffungskosten nicht in die Kraftstoffleitung miteinbezogen. Diesbezüglich ist beispielsweise aus der US 2005/132779 A1 ein Laborverfahren zur Bestimmung der Viskosität von Wasser-in-Öl-Emulsionen bekannt

Daneben ist es bekannt, die Qualität von einer Verbrennungsmaschine zugeführtem Öl und dessen Kontamination mit Kraftstoff durch das in der US 2002/0011095 A1 vorgestellte Verfahren, mit dem die Viskosität des Öls gemessen wird, zu überprüfen.

Aufgabe der Erfindung ist es, ein Meßverfahren zur Bestimmung der Viskosität von Kraftstoff/Wasser- und Wasser/Kraftstoff-Emulsionen in Kraftstoffleitungen bereit zu stellen, das mit den bekanten Mitteln eine exakte Bestimmung der Viskosität dieser Emulsionen erlaubt.

Die Aufgabe wird erfindungsgemäß durch die Merkmale des Verfahrens von Anspruch 1 gelöst. Die Unteransprüche geben vorteilhafte Ausgestaltungen der Erfindung wieder.

Wie oben dargestellt wurde festgestellt, dass bei der Messung der Viskosität von Kraftstoff/Wasser- und Wasser/Kraftstoff-Emulsionen erhebliche Meßfehler auftreten. Weiter wurde festgestellt, dass das Ausmaß des Fehlers dabei vom Wassergehalt der Emulsion abhängig ist.

Grundgedanke der Erfindung ist es nun, die (wahre) Viskosität einer Kraftstoff/Wasser- oder Wasser/Kraftstoff-Emulsion dadurch zu bestimmen, dass der für eine bestimmte Emulsion von einem Viskosimeter unter Verwendung eines ersten (ungenauen) Messverfahrens erfasste Viskositätswert automatisch korrigiert wird.

Dieses erfolgt dadurch, dass zunächst unabhängig von dem ersten Messverfahren die wahre (bzw. tatsächliche) Viskosität mehrerer Kraftstoff/Wasser- oder Wasser/Kraftstoff-Emulsionen mit unterschiedlichem Wasseranteil mit einem geeigneten zweiten Messverfahren mit höherer Messgenauigkeit (und gegebenenfalls in Abhängigkeit von der Temperatur) unter Laborbedingungen experimentell exakt bestimmt wird und den mit einem in Kraftstoffleitungen herkömmlich verwendeten System mittels des ersten ungenaueren Messverfahrens bestimmten Viskositätswerten zugeordnet wird. Bevorzugt wird die tatsächliche Viskosität dabei mit einem Rotationsviskosimeter oder einem Rheometer bestimmt.

Bei Messung eines Viskositätswerts mittels des ersten Verfahrens in einer Kraftstoffleitung kann so durch beispielsweise tabellarische Zuordnung oder soweit möglich durch Verwendung von Korrekturfaktoren auf die tatsächliche Viskosität der Emulsion (also den experimentell mittels des zweiten Verfahrens exakt bestimmten Viskositätswert) geschlossen werden. Erfindungsgemäße Voraussetzung hierfür ist, dass das zweite Meßverfahren gegenüber dem ersten Meßverfahren genauer ist, also weniger fehlerbehaftet ist. Die Vorzüge einzelner Messverfahren und Apparate sind dem Fachmann entweder bekannt oder er kann diese nun einfach durch Vergleich von Viskositätsmessungen von Kraftstoff/Wasser- und Wasser/Kraftstoff-Emulsionen leicht vermitteln.

Erfindungsgemäß ist es zur Zuordnung der tatsächlich vorliegenden Viskosität lediglich notwendig, den Wassergehalt der Emulsion in der Kraftstoffleitung zu bestimmen, um die richtige Zuordnung von gemessenem zu wahrem Viskositätswert vornehmen zu können.

Dieses erfolgt bevorzugt über die Messung des kapazitiven Widerstands der jeweils in der Kraftstoffleitung geförderten Kraftstoff/Wasser- oder Wasser/Kraftstoff-Emulsion. Beispielsweise wird dabei auf einen bekannten Aufbau von zwei koaxial ausgerichteten Rohrleitungen mit einem inneren und einem äußeren Rohr zurückgegriffen, wobei das innere Rohr die zu untersuchende Flüssigkeit, also den Kraftstoff oder Emulsionskraftstoff, führt. Indem ein sinusförmiges Signal mit bekannter Amplitude auf das innere Rohr gegeben wird, kann der kapazitive Widerstand der Flüssigkeit gemessen werden.

Da die Messung des kapazitiven Widerstands allerdings nur in Abhängigkeit der Länge des Messrohrs in jeweils einem schmalen Teilbereich lineare Antworten produziert, ist bevorzugt vorgesehen, dass zwischen mehreren Sensoren mit unterschiedlich langen Messrohren ausgewählt werden kann, wobei dasjenige Messsignal zur Bestimmung ausgewählt wird, das im linearen Messbereich des jeweiligen Sensors liegt.

Schließlich wird auch eine Kraftstoffleitung beansprucht, die einen in der Kraftstoffleitung angeordneten die Viskosität des Kraftstoffs messenden ersten Sensor, eine Wärmeeinrichtung zum Erwärmen und/oder Kühlen des Kraftstoffs und eine mit dem Sensor und der Wärmeeinrichtung verbundene Steuerung zum Einstellen einer vorbestimmten Viskosität aufweist. Diese enthält erfindungsgemäß weiter einen den Wassergehalt des Kraftstoffs messenden zweiten Sensor zum Bestimmen des Wassergehalts des Kraftstoffs, wobei auch der zweite Sensor mit der Steuerung verbunden ist und die Steuerung zum Bestimmen der Viskosität des Kraftstoffs durch Zuordnen des mit dem ersten Sensor gemessenen Werts zu einem mit einem dritten Sensor vorbestimmten Viskositätswert für einen Kraftstoff mit einem dem gemessenen Wassergehalt entsprechenden Wassergehalt und zum Steuern der Wärmeeinrichtung in Abhängigkeit vom zugeordneten Viskositätswert eingerichtet ist.

Bevorzugt misst der zweite Sensor - wie oben genannt - den kapazitiven Widerstands des Kraftstoffs. Besonders bevorzugt ist eine Mehrzahl von zweiten Sensoren mit unterschiedlichen linearen Messbereichen vorgesehen ist, wobei die Steuerung für die Bestimmung des Wassergehalts des Kraftstoffs zum Auswählen desjenigen zweiten Sensors eingerichtet ist, dessen Messwert im linearen Messbereich liegt.

Auch hier gilt entsprechend, dass der dritte Sensor eine höhere Messgenauigkeit als der erste Sensor aufweist, wobei die beispielsweise tabellarische Zuordnung über einen Speicher, der die mit dem dritten Sensor vorbestimmten Viskositätswerte für die Steuerung zugreifbar abspeichert, erfolgt. Der dritte Sensor wird daher in der Regel ein anderes Messverfahren als der erste Sensor verwenden (siehe oben).

Es versteht sich, dass nicht alle Viskositäten für alle Mischungsverhältnisse von Wasser zu Kraftstoff in Kraftstoff/Wasser- und Wasser/Kraftstoff-Emulsionen vorab bestimmt werden können, sodass zum Bestimmen der wahren Viskosität mathematische Interpolationsverfahren auf der Grundlage der vorbestimmten Viskositätswerte angewendet werden können, um eine zumindest näherungsweise exakte Zuordnung der gemessenen Viskosität zur wahren Viskosität vornehmen zu können. Dieser interpolierte Wert ist in jedem Fall viel genauer als der in der Kraftstoffleitung gemessene Wert.

## Patentansprüche

1. Verfahren zur Bestimmung der Viskosität einer Kraftstoff/Wasser- oder Wasser/Kraftstoff-Emulsion in einer Kraftstoffleitung mit dem Schritt:
- Messen der Viskosität der Emulsion mit einem ersten Messverfahren,
**gekennzeichnet durch** die weiteren Schritte:
- Messen des Wassergehalts der Emulsion,
- Bestimmen der Viskosität der Emulsion **durch** Zuordnen des mit dem ersten Messverfahren gemessenen Werts zu einem mit einem zweiten Messverfahren vorbestimmte Wert für eine Emulsion mit einem dem gemessenen Wassergehalt entsprechenden Wassergehalt, wobei das Zuordnen tabellarisch oder mittels eines Korrekturfaktors erfolgt,
wobei die Viskosität mit dem zweiten Messverfahren mit gegenüber dem ersten Messverfahren höherer Meßgenauigkeit unter Laborbedingungen experimentell exakt bestimmt wird.

2. Verfahren nach Anspruch 1, **dadurch gekennzeichnet, dass** das Messen der Viskosität der Emulsion mit dem ersten Meßverfahren das Messen der Schwingungsdämpfung eines mit höher Frequenz und geringer Amplitude angeregten Messkörpers beinhaltet.

3. Verfahren nach einem der vorhergehenden Ansprüche, **dadurch gekennzeichnet, dass** das Messen der Emulsion nach dem zweiten Messverfahren mit einem Rheometer erfolgt.

4. Verfahren nach einem der vorhergehenden Ansprüche, **dadurch gekennzeichnet, dass** der Wassergehalt der Emulsion durch Bestimmen des kapazitiven Widerstands der Emulsion erfolgt.

5. Verfahren nach einem der vorhergehenden Ansprüche, **dadurch gekennzeichnet, dass** das Bestimmen der Viskosität mittels Interpolation der vorbestimmten Werte erfolgt.

6. Kraftstoffleitung mit einem in der Kraftstoffleitung angeordneten die Viskosität des Kraftstoffs messenden ersten Sensor, einer Wärmeeinrichtung zum Erwärmen und/oder Kühlen des Kraftstoffs und einer mit dem Sensor und der Wärmeeinrichtung verbundenen Steuerung zum Einstellen einer vorbestimmten Viskosität, **gekennzeichnet durch**
einen den Wassergehalt des Kraftstoffs messenden zweiten Sensor zum Bestimmen des Wassergehalts des Kraftstoffs,
wobei der zweite Sensor mit der Steuerung verbunden ist und die Steuerung zum Bestimmen der Viskosität des Kraftstoffs **durch** Zuordnen des mit dem ersten Sensor gemessenen Werts zu einem mit einem Messgerät mit gegenüber dem ersten Sensor höherer Messgenauigkeit vorbestimmten Viskositätswert für einen Kraftstoff mit einem dem gemessenen Wassergehalt entsprechenden Wassergehalt und zum Steuern der Wärmeeinrichtung in Abhängigkeit vom zugeordneten Viskositätswert eingerichtet ist, wobei das Zuordnen tabellarisch oder mittels eines Korrekturfaktors erfolgt,

7. Kraftstoffleitung nach Anspruch 6, **dadurch gekennzeichnet, dass** der zweite Sensor zum Messen des kapazitiven Widerstands des Kraftstoffs eingerichtet ist.

8. Kraftstoffleitung nach einem der Anspruche 6 und 7, **dadurch gekennzeichnet, dass** eine Mehrzahl von zweiten Sensoren mit unterschiedlichen linearen Messbereichen vorgesehen ist und die Steuerung für die Bestimmung des Wassergehalts des Kraftstoffs zum Auswählen desjenigen zweiten Sensors eingerichtet ist, dessen Messwert im linearen Messbereich liegt.

## Claims

1. A method for determining the viscosity of a fuel/water or a water/fuel emulsion in a fuel line having the step:
- measuring the viscosity of the emulsion using a first measurement method, **characterized by** the further steps:
- measuring the water content of the emulsion,
- determining the viscosity of the emulsion by assigning the measured value measured using the first measurement method to a value, predetermined using a second measurement method, for an emulsion having a water content corresponding to the measured water content, assigning taking place in table form or by means of a correction factor,
the viscosity being accurately determined experimentally under laboratory conditions using the second measurement method having a higher measuring accuracy relative to the first measurement method.

2. The method according to Claim 1, **characterized in that** measuring the viscosity of the emulsion using the first measurement method involves measuring the vibration damping of a measuring body excited at a high frequency and a small amplitude.

3. The method according to one of the preceding claims, **characterized in that** measuring the emulsion according to the second measurement method takes place using a rheometer.

4. The method according to one of the preceding claims, **characterized in that** determining the water content of the emulsion takes place by determining the capacitive resistance of the emulsion.

5. The method according to one of the preceding claims, **characterized in that** determining the viscosity takes place by means of interpolation of the predetermined values.

6. A fuel line having a first sensor arranged in the fuel line and measuring the viscosity of the fuel, a heating device for heating and/or cooling the fuel and a control system, connected to the sensor and the heating device, for adjusting a predetermined viscosity,
**characterized by**
a second sensor measuring the water content of the fuel for determining the water content of the fuel,
the second sensor being connected to the control system and the control system being designed for determining the viscosity of the fuel by assigning the value measured using the first sensor to a viscosity value predetermined using a measurement device having a higher measuring accuracy than the first sensor, for a fuel having a water content corresponding to the measured water content, and for controlling the heating device as a function of the assigned viscosity value, assigning taking place in table form or by means of a correction factor.

7. The fuel line according to Claim 6, **characterized in that** the second sensor is designed for measuring the capacitive resistance of the fuel.

8. The fuel line according to one of Claims 6 and 7, **characterized in that** a plurality of second sensors having differing linear measuring ranges is envisaged and the control system is designed for determining the water content of the fuel for choosing that second sensor whose measured value is within the linear measuring range.

## Revendications

1. Procédé permettant de déterminer la viscosité d'émulsions carburant carburant/eau et eau-carburant par le procédé suivant :
- Mesure de la viscosité de l'émulsion au moyen d'un premier procédé de mesure,
**caractérisé par** les étapes suivantes :
- Mesure de la teneur en eau de l'émulsion,
- Détermination de l'émulsion en attribuant la valeur mesurée lors du premier procédé de mesure à une valeur prédéterminée lors d'un deuxième procédé de mesure pour une émulsion ayant la teneur en eau correspondant à la valeur mesurée, à savoir que cette attribution se fait au moyen d'un tableau ou d'un facteur de correction,
à savoir qu'avec le deuxième procédé de mesure dans des conditions expérimentales de laboratoire, la viscosité est déterminée avec une plus grande précision qu'avec le premier procédé de mesure.

2. Procédé selon la revendication 1, **caractérisé en ce que** la mesure de la viscosité de l'émulsion au moyen du premier procédé de mesure comprend la mesure de l'amortissement des vibrations d'un corps de mesure stimulé par haute fréquence et faible amplitude.

3. Procédé selon une des revendications précédentes, **caractérisé en ce que** la mesure de l'émulsion a lieu après le deuxième procédé de mesure, au moyen d'un rhéomètre.

4. Procédé selon une des revendications précédentes, **caractérisé en ce que** la teneur en eau de l'émulsion est déterminée en contrôlant la résistance capacitative de l'émulsion.

5. Procédé selon une des revendications précédentes, **caractérisé en ce que** détermination de la viscosité se fait au moyen d'une interpolation des valeurs prédéterminées.

6. Conduite de carburant équipée d'un premier capteur installé en son intérieur et mesurant la viscosité du carburant, d'un dispositif de chauffe permettant de chauffer et/ou de refroidir le carburant, et d'un dispositif de commande relié au capteur et au dispositif de chauffe et permettant de prérégler la viscosité.
**caractérisé par**
un deuxième capteur mesurant la teneur en eau du carburant afin de déterminer la teneur en eau du carburant,
à savoir que le deuxième capteur est relié au dispositif de commande et que ledit dispositif de commande est réglé pour déterminer la viscosité du carburant en attribuant la valeur mesurée avec le premier capteur à une valeur de viscosité, prédéterminée au moyen d'un appareil de mesure, et avec plus grande précision de mesure qu'avec le premier capteur, pour un carburant dont la teneur en eau correspond à la teneur en eau mesurée et pour régler la chaleur en fonction de la valeur de viscosité qui lui a été attribuée, à savoir que l'attribution se fait par un tableau ou au moyen d'un facteur de correction.

7. Conduite de carburant selon la revendication 6, **caractérisée en ce que** le deuxième capteur est réglé pour mesurer la résistance capacitative du carburant.

8. Conduite de carburant selon les revendications 6 et 7, **caractérisée en ce qu'**une multitude de deuxièmes capteurs sont prévus avec différenties fourchettes de mesure linéaire, et que le dispositif de commande est réglé pour déterminer la teneur en eau du carburant, afin de permettre de choisir le deuxième capteur dont la valeur mesurée se situe dans une fourchette de mesure linéaire.
